# EUROPEAN PATENT SPECIFICATION

(11) **EP 0 299 338 B1**
(45) Date of publication and mention of the grant of the patent: **06.10.1993**
(21) Application number: 88110738.7
(22) Date of filing: 05.07.1988
(51) Int. Cl.: A61N 1/39, A61N 1/08

(54) **Defibrillator**
Defibrillator
Défibrillateur

(30) Priority: 11.07.1987 JP 106745/87
(43) Date of publication of application: 18.01.1989
(73) Proprietor: Nihon Kohden Corporation, Shinjuku-ku Tokyo (JP)
(72) Inventor: Miyajima, Nobukazu, Tokorozawa-shi Saitama-ken (JP); Tanaka, Akira, Okegawa-shi Saitama-ken (JP); Shinoda, Masaru, Kita-ku Tokyo (JP)
(74) Representative: MEISSNER, BOLTE & PARTNER

(56) References cited:
- DE-A- 2 612 768
- MEDICAL AND BIOLOGICAL ENGINEERING, vol. 13, no. 2, March 1975, pages 240-244; J.W.MACHIN: "A portable mains-operated d.c. defibrillator of unusual design"

## Description

### BACKGROUND OF THE INVENTION

The present invention relates to a defibrillator in which a high-voltage generating circuit charges a high-voltage capacitor circuit with a high voltage, and which is adapted to apply the charged high voltage by an electrode or a paddle to a body of a patient by turning on or closing an output switch.

In a conventional apparatus of this type, such a high-voltage capacitor circuit is formed by a capacitor in which the electrodes are of a metal-film type and the terminals have a tab structure with which lead wires can be inserted. Accordingly, such a capacitor tends to be bulky as a high-voltage capacitor and raises problems concerning its durability because the lead wires are subjected to stress during the discharge of large-magnitude currents.

From the document "A portable mains-operated d.c. defibrillator of unusual design", published by J.W. Machin in Medical and Biological Engineering, Vol. 13, No. 2, March 1975, pages 240 to 244, a conventional defibrillator is known comprising a voltage sensor including two transistors in a Darlington connection forming a Schmitt-trigger-circuit with a third transistor. A capacitor bank is provided utilizing electrolytic capacitors. Resistors and zener diodes are provided in order to supply different discrete energy levels so that the defibrillator is calibrated for use. In this conventional device, the capacitor circuit is not provided with any metallized plastic film capacitor, rather electrolytic capacitors are used. Moreover, no means are provided in order to detect any charge abnormalities. Therefore, it is not possible to inhibit the further supply of any high voltage when any charge abnormality has occurred.

In order to solve these problems, one may alternatively use a metallized plastic film capacitor. In this case, however, one has to cope with the problem that such a capacitor may have a self-healing ability. That is, because of this self-healing ability, if a dielectric breakdown takes place in the high-voltage capacitor circuit, the capacitor does not become unusable automatically and can be charged again with the high voltage which, however, is lower than a prescribed and necessary high voltage. The capacitor is thus maintained in a usable condition but it remains unstable.

In a conventional metallized plastic film capacitor, the electrodes are composed of deposited layers and the lead wires extend from metallic contact or metal sprayed portions at both ends of wound-up films. Although this arrangement makes it possible to reduce the tan δ value, it raises another problem in that, if a dielectric breakdown takes place, a short- circuiting current may flow locally, producing a loud explosion noise.

### SUMMARY OF THE INVENTION

In view of the above-described circumstances, the object underlying the present invention is to provide a defibrillator which is more reliable in operation and capable of automatically detecting a dielectric breakdown of the high-voltage capacitor circuit and of stopping operation of the device.

Another object of the present invention is to provide a high-voltage capacitor circuit comprising a metallized plastic film capacitor which is very suitable for use in a defibrillator.

According to the present invention, in a defibrillator having various circuit structures known per se, a high-voltage capacitor circuit consists of a metallized plastic film capacitor. Further components of the defibrillator are a charge abnormality detecting circuit for detecting a sharp increase in charge current and/or a sharp drop in charge voltage which are caused by a dielectric breakdown of the metallized plastic film capacitor, a notifying means for notifying any abnormality in response to a detection signal of the detecting circuit, and a switch circuit for inhibiting the further supply of a high voltage to the high-voltage capacitor circuit, namely in response to the detection signal, either by blocking the voltage input to or the output from the high-voltage generating circuit or by stopping the operation of the high-voltage generating circuit.

This arrangement makes it possible to detect and notify any dielectric breakdown of the high-voltage capacitor circuit. Therefore, even if a metallized plastic film capacitor is used in the high-voltage capacitor circuit, it is possible to eliminate the prior art problem that, on account of the self-healing characteristics, the capacitor is re-charged with an insufficiently high voltage and is then operated in its unstable condition.

Further, the metallized plastic film capacitor circuit comprises a plurality of capacitor elements each of which have metallized plastic films wound up, and metallic contacts provided on the end surfaces on both sides thereof. Resistors each of which have a resistance at least equal to the internal resistance of each capacitor element per se are connected to metallic contact portions of the capacitors elements.

By virtue of this arrangement, the high-voltage capacitor circuit for the defibrillator is made compact. Further, since the capacitor circuit is divided into a plurality of capacitor elements, the number of times the thin metal layers must be wound is reduced, thereby facilitating manufacture while improving the reliability after manufacture.

Still further, in the event of dielectric breakdown any short-circuiting discharges from capacitor elements are allowed to take place via the additional resistors, thereby avoiding any localized discharge, and thereby reducing any considerable level of explosion noise.

The defibrillator according to the invention comprises:
- a high-voltage generating circuit;
- a high-voltage capacitor circuit which is adapted to be charged with a high voltage by the high-voltage generating circuit and which consists of a metallized plastic film capacitor;
- an output circuit which includes at least a switch and is adapted to apply the high voltage of the high-voltage capacitor circuit to paddle electrodes to be brought in contact with a body of a patient;
- a charge abnormality detecting circuit for detecting a sharp increase in charge current and/or a sharp drop in charge voltage which are caused by a dielectric breakdown of the metallized plastic film capacitor;
- a notifying means for notifying any abnormality in response to a detection signal of the detecting circuit; and
- a switch circuit for inhibiting the further supply of any high voltage from the high voltage generating circuit in response to the detection signal.

According to a specific embodiment of the defibrillator according to the invention, the charge abnormality detecting circuit comprises
- a voltage divider for the high voltage supplied by the high-voltage capacitor circuit,
- a peak holding circuit to which a fraction voltage of the voltage divider is input, and
- a comparator for comparing the fraction voltage with a predetermined voltage lower than the holding voltage of the peak holding circuit and for generating an alarm and inhibiting signal when the present fraction voltage is below the predetermined voltage.

According to another specific embodiment of the defibrillator according to the invention, the charge abnormality detecting circuit comprises
- an A/D converter for digitizing a fraction voltage of the high-voltage capacitor circuit,
- a memory storing data of curves representing the normal charging and discharging operation of the high-voltage capacitor circuit, and
- a CPU for continuously comparing the digitized fraction voltage with the stored charging and discharging curves and for generating an alarm and inhibiting signal if the deviation exceeds values of normal operation by a predetermined amount.

According to a further development of the defibrillator according to the invention the metallized plastic film capacitor comprises
- a plurality of capacitor elements each of which has metallized plastic films wound up and metallic contacts provided on the end surfaces on both sides thereof and which are received in a common case,
- a pair of capacitor terminals which extend from the case and which are connected to the metallic contact portions of the capacitor elements provided on the end surfaces on both sides thereof, and
- resistors each of which have a resistance at least equal at least equal to the internal resistance of the respective capacitor elements per se and which are connected to the metallic contact portions that are provided on the end surfaces on at least one side of the capacitor elements.

According to a further development of the defibrillator according to the invention, the resistors are connected each between one of the pair of capacitors terminals and each of the metallic contact portions that are provided on the end surfaces on one side of the capacitor elements.

According to a still further development of the defibrillator according to the invention, the metallic contact portions that are provided on the end surfaces on one side of the capacitor elements are connected in sequence through the resistors.

The invention will be explained in more detail hereinafter by means of various preferred embodiments and with reference to the accompanying drawings.

### BRIEF DESCRIPTION OF THE DRAWINGS

Fig. 1 is a diagram showing the basic circuit arrangement of a defibrillator in accordance with the present invention;
Fig. 2 is a diagram showing a circuit arrangement of a defibrillator in accordance with a first embodiment of the present invention;
Fig. 3 is an illustration schematically showing the arrangement of a high-voltage capacitor circuit of the defibrillator;
Fig. 4 is a diagram showing a circuit arrangement of a defibrillator in accordance with another embodiment of the present invention;
Fig. 5 is a partially-broken perspective view of a high-voltage capacitor circuit for a defibrillator in accordance with a further embodiment of the present invention;
Fig. 6 is a perspective view of metallized plastic films of the capacitor circuit in accordance with the embodiment shown in Fig. 5; and
Fig. 7 is an illustration schematically showing the arrangement of a high-voltage capacitor circuit in accordance with a still further embodiment of the present invention.

### DESCRIPTION OF THE PREFERRED EMBODIMENTS

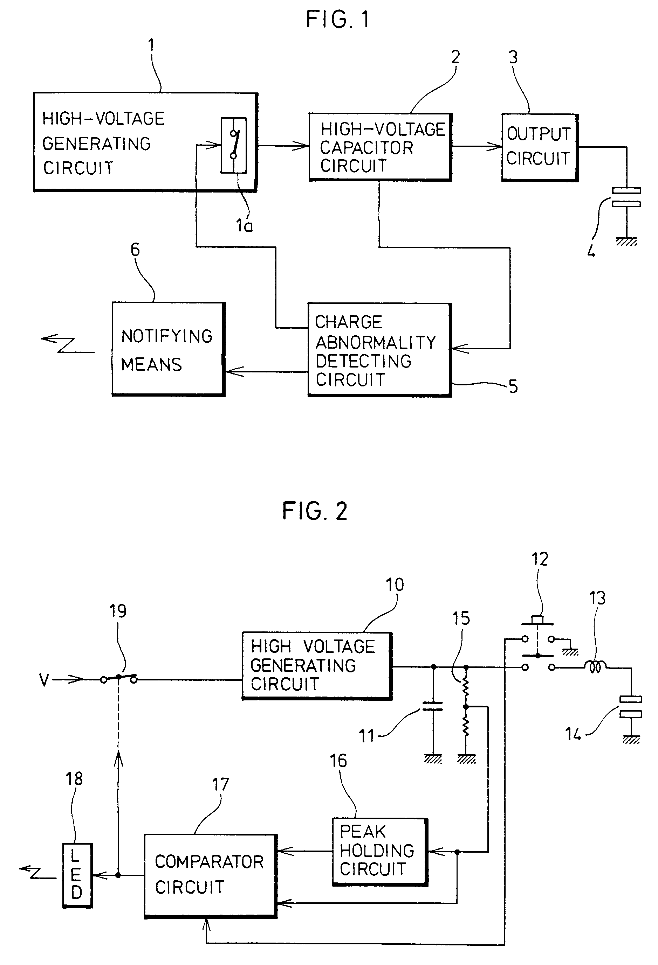

Referring to Fig. 1, a defibrillator which is known in principle is composed of a high-voltage generating circuit 1, a high-voltage capacitor circuit 2 adapted to be charged with a high-voltage by the high-voltage generating circuit 1, and an output circuit 3 including at least a switch for applying a high voltage to electrodes in particular paddle electrodes 4 to be brought in contact with a body of a patient. Various structures may be used for each of the respective components as explained hereinafter.

According to the present invention, the high-voltage capacitor circuit 2 comprises a metallized plastic film capacitor. The basic circuit arrangement according to Fig. 1 further comprises a charge abnormality detecting circuit 5 connected to the high-voltage capacitor circuit 2 and to a switch circuit 1a of the high-voltage generating circuit 1 for detecting a sharp increase of the charge current and/or a sharp drop of the charge voltage which are caused by a dielectric breakdown of the metallized plastic film capacitor, and indicating or notifying means 6 connected to the charge abnormality detecting circuit 5 for notifying any abnormality in response to a detection signal of the detecting circuit 5 by emitting light and/or sound.

The switch circuit 1a is provided for inhibiting the feeding of a high voltage to the high-voltage capacitor circuit 2 in response to a corresponding detection signal from the detecting circuit 5 by blocking the voltage input to or the output from the high-voltage generating circuit 1 or by stopping the operation of the high-voltage generating circuit 1.

Once a dielectric breakdown takes place in the metallized plastic film capacitor serving as the high-voltage capacitor circuit 2, the charge abnormality detecting circuit 5 detects an abnormality in charging during a charging process or in the charged condition after the completion of charging. By this detection, the switch circuit 1a is operated and inhibits the feeding of a high voltage to the metallized plastic film capacitor, thereby preventing the capacitor from being re-charged by its self-healing ability.

Simultaneously, the notifying means 6 notifies the dielectric breakdown of the capacitor circuit 2 to the operator, thus enabling the capacitor circuit to be replaced, and thereby eliminating the risk that a self-healed capacitor is used furtheron in its condition having a reduced capacity.

Fig. 2 shows a circuit arrangement of a defibrillator in accordance with a second and specific embodiment of the present invention.

Referring to Fig. 2, the arrangement of a defibrillator is such that a high-voltage capacitor circuit 11 is charged by a high-voltage generating circuit 10, and, when a switch 12 is turned on or closed, the charge voltage of the high-voltage capacitor circuit 11 is applied to an electrode in particular a paddle electrode 14 via an inductance 13.

According to the present invention, the defibrillator is provided with a charge abnormality detecting circuit. This charge abnormality detecting circuit comprises a voltage divider 15 connected in parallel to the high-voltage capacitor circuit 11, a peak holding circuit 16 to which a fraction voltage of the voltage divider 15 is input, and a comparator circuit 17. The inputs of a comparator 17 are connected to the output of the peak holding circuit 16 and to the input of the peak holding circuit 16 supplied with a signal from the tap of the voltage divider 15. Also, the comparator circuit 17 receives a control signal from a switch 12 connected between the high-voltage generating circuit 10 and the inductance 13.

The holding voltage of the peak holding circuit 16 is input to the comparator circuit 17 which is adapted to generate a signal when the fraction voltage of the voltage divider 15 drops below a predetermined value, for example 3/4 of the holding voltage. The output of the comparator circuit 17 is supplied to an LED 18 for indicating or notifying any abnormality, and simultaneously it is used to drive a switch 19 for blocking the voltage input to the high-voltage generating circuit 10.

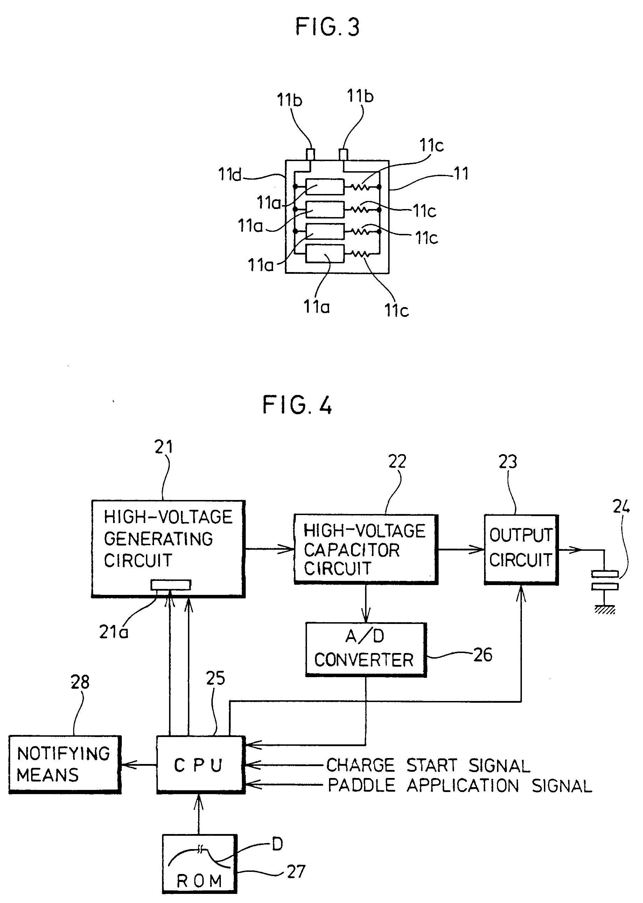

As shown in Fig. 3, the high-voltage capacitor circuit 11 has a plurality of capacitor elements 11a received in a common case 11d. Each of the capacitor elements 11a is formed of metallized plastic films wound up and is provided with metallic contacts at the ends on both sides thereof. The metallic contact portions at the ends on either side of the capacitor elements 11a are connected to either one of a pair of capacitor terminals 11b extending from the case 11d.

All the capacitor elements 11a are connected in parallel to each other and connected between the pair of capacitor terminals 11b. The arrangement of this capacitor circuit 11 is such that a plurality of resistors 11c are provided each of which has at least the same resistance as the internal resistance of each capacitor element 11a per se, and are interposed in series each between the metallic contact portions provided on the ends on one side of the respective capacitor elements 11a and the corresponding terminal 11b.

The comparator circuit 17 is kept inoperative during a discharging period so as to prevent the comparator circuit 17 from being erroneously actuated by a drop in voltage during the discharge of the charge voltage via the electrode or the paddle electrode 14 to the body of a patient when the switch 12 has been closed.

The operation of defibrillator is as follows:
The voltage of the capacitor circuit 11 rises with a saw-tooth waveform while the capacitor circuit 11 is being charged so that the holding voltage of the peak holding circuit 16 is sequentially regenerated and the peak holding 16 holds a voltage corresponding to the charge voltage. Therefore, during a charging period, signals input to the terminals of the comparator circuit 17 are at substantially the same level and the comparator circuit 17 generates no signal.

When the switch 12 is closed or turned on after the completion of charging, the charge voltage is discharged to the body of a patient via corresponding electrodes namely the so-called paddle electrodes 14. During this discharging period, a control signal is supplied from the switch 12 to the comparator circuit 17 so that the comparator circuit 17 will not detect any difference from the holding voltage.

However, if a dielectric breakdown takes place either during the charging of the capacitor 11 before the closing or turning-on of the switch 12 or after the completion of charging, the dielectric breakdown causes a sharp drop in the charge voltage so that the fraction voltage at the voltage divider is reduced to a value which is lower than a predetermined value, for example lower than a 3/4-value of the peak-holding voltage.

Then, the comparator circuit 17 outputs a detection signal. In response to this detection signal, the LED 18 is turned on to notify the dielectric breakdown of the capacitor circuit 11 and, simultaneously, the switch 19 is operated to interrupt the generation of a high voltage. In this way, the capacitor circuit 11 is positively prevented from being re-charged due to its self-healing characteristics.

The capacity of the capacitor circuit 11 corresponds to that of the capacitor elements 11a connected in parallel. By virtue of the interposition of the resistors 11c, the short-circuiting flow of current from one capacitor element 11a to others due to the breakdown can be controlled by corresponding time constants, thereby reducing the level of any explosion noises.

In the foregoing embodiment, the charge abnormality detecting circuit may alternatively be such that the capacitor circuit 11 is connected to, instead of the voltage divider 15, serially connected resistors having a small resistance which are provided for detecting the charge current, wherein a differentiation circuit is adapted to detect a sharp increase in current.

Fig. 4 illustrates another embodiment of the present invention. A defibrillator comprises a high-voltage generating circuit 21, a high-voltage capacitor circuit 22, an output circuit 23, an electrode or a paddle electrode 24, and a CPU 25 provided for control purposes. According to this embodiment, the CPU 25 is used in the following manner. A fraction of a high voltage which has been digitized by an A/D converter 26 is fed to the CPU 25 to be compared, as time passes by, with data D of curves which are stored in a ROM 27 expressing and corresponding to the operation of normal charging and discharging (to a patient).

If any change in the charge voltage occurs and the charge voltage deviates from the charging and discharging curve data D by a large extent, a detection is effected detecting a dielectric breakdown. The detection of such a dielectric breakdown is also possible during the discharge of the charge voltage to the body of a patient. When this detection is effected, operations similar to those described above are performed, that is, a switch 21a inhibits the further supply of a high voltage by the high-voltage generating circuit 21, while a notifying means 28 notifies the dielectric breakdown.

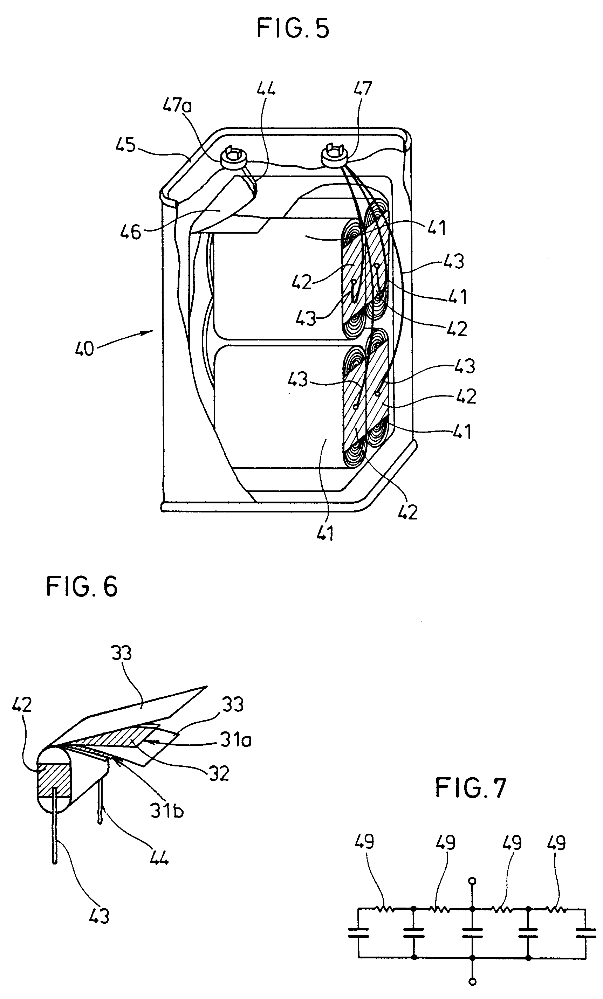

Fig. 5 shows a winding structure of a high-voltage capacitor circuit 40 in accordance with the present invention, the capacitor circuit 40 being similar to the above-described high-voltage capacitor circuit 11.

The capacitor circuit 40 comprises, for instance, four capacitor elements 41 each of which are formed by winding up metallized plastic films 31a and 31b on which layers 32 of a metal, e.g. aluminum, are deposited, with protective films 33 being inserted between the films, in such a manner as to provide non-deposited portions at both lateral ends of the capacitor element (see Fig. 6).

Metallic contacts 42 are provided on the end surfaces of each capacitor element 41 on both sides thereof. Resistance wires 43 are soldered to the end surfaces of the capacitor elements 41 on one side, while a lead wire 44 is soldered to the end surfaces on the other side so as to connect the capacitor elements 41 in parallel.

All the four capacitor elements 41 are surrounded by a pressed board 46 and are sealed within a case 45 filled with insulating oil. The resistance wires 43 are connected to one terminal 47 of a pair of terminals 47, 47a formed on the upper surface of the case 45, while the single lead wire 44 extending from the junctions of the parallel connection is connected to the other terminal 47a.

Each of the capacitor elements 41 has a capacity of, for instance, 10 µF, a dielectric strength of 10 kV, and an internal resistance between the metallic contacts 42 at the end surfaces, which is about several Ohms and is smaller than the resistance of the body of a patient to be treated during the discharge, that is about 50 Ohms. The resistance of each resistance wire 43 is set at substantially the same level as that of the above-mentioned internal resistance of each capacitor element 41.

In the foregoing embodiments, the number of the capacitor elements can be set at any value in accordance with the capacity, the ease of manufacturing, the allowable level of any explosion noise, etc. The additional resistors may alternatively be interposed between the metallic contacts provided on the end surfaces on both sides of the capacitor elements. With such an arrangemenet in which the metallic contacts are connected in sequence via the additional resistors 49, it is possible to make the number of the additional resistors smaller by one than the number of the capacitor elements, as shown in Fig. 7.

The resistance of each interposed resistor 49 is required to be at least equal to the internal resistance of each capacitor element, from the viewpoint of reducing the level of any explosion noise. This resistance may be set to a larger value if it does not cause problems concerning energy loss and the time constant during the discharge via the paddle electrode 24. The present invention may be used with metallized plastic films of another type, such as the one-surface deposit type as well.

## Claims

1. A defibrillator comprising:
- a high-voltage generating circuit (1, 10, 21);
- a high-voltage capacitor circuit (2, 11, 22, 40) which is adapted to be charged with a high voltage by the high-voltage generating circuit (1, 10, 21) and which consists of a metallized plastic film capacitor (11, 40);
- an output circuit (3, 13, 23) which includes at least a switch (12) and is adapted to apply the high voltage of the high-voltage capacitor circuit (2, 11, 22, 40) to paddle electrodes (4, 14, 24) to be brought into contact with a body of a patient;
- a charge abnormality detecting circuit (5, 15-17, 25-27) for detecting a sharp increase in charge current and/or a sharp drop in charge voltage which are caused by a dielectric breakdown of the metallized plastic film capacitor (11, 40);
- a notifying means (6, 18, 28) for notifying any abnormality in response to a detection signal of the detecting circuit (5, 15-17, 25-27); and
- a switch circuit (1a, 19, 21a) for inhibiting the further supply of any high voltage from the high-voltage generating circuit (1, 10, 21) in response to the detection signal.

2. The defibrillator according to claim 1,
wherein the charge abnormality detecting circuit (15-17) comprises
- a voltage divider (15) for the high voltage supplied by the high-voltage capacitor circuit (10),
- a peak holding circuit (16) to which a fraction voltage of the voltage divider (15) is input, and
- a comparator (17) for comparing the fraction voltage with a predetermined voltage lower than the holding voltage of the peak holding circuit (16) and for generating an alarm and inhibiting signal when the present fraction voltage is below the predetermined voltage.

3. The defibrillator according to claim 1,
wherein the charge abnormality detecting circuit (25-27) comprises
- an A/D converter (26) for digitizing a fraction voltage of the high-voltage capacitor circuit (22),
- a memory (27) storing data of curves representing the normal charging and discharging operation of the high-voltage capacitor circuit (22), and
- a CPU (25) for continuously comparing the digitized fraction voltage with the stored charging and discharging curves and for generating an alarm and inhibiting signal if the deviation exceeds values of normal operation by a predetermined amount.

4. The defibrillator according to any of claims 1 to 3,
wherein the metallized plastic film capacitor comprises
- a plurality of capacitor elements (11a, 41) each of which have metallized plastic films (31a, 31b) wound up and metallic contacts (42) provided on the end surfaces on both sides thereof and which are received in a common case (45),
- a pair of capacitor terminals (11b, 47, 47a) which extend from the case (45) and which are connected to the metallic contact portions (42) of the capacitor elements (11a, 41) provided on the end surfaces on both sides thereof, and
- resistors (11c, 43) each of which have a resistance at least equal to the internal resistance of the respective capacitor elements (11a, 41) per se and which are connected to the metallic contact portions (42) that are provided on the end surfaces on at least one side of the capacitor elements (11a, 41).

5. The defibrillator according to claim 4,
wherein the resistors (11c, 43) are connected each between one (11b, 47) of the pair of capacitor terminals (11b, 47, 47a) and each of the metallic contact portions (42) that are provided on the end surfaces on one side of the capacitor elements (11a,41).

6. The defibrillator according to claim 4 or 5,
wherein the metallic contact portions that are provided on the end surfaces on one side of the capacitor elements are connected in sequence through the resistors (49).

## Patentansprüche

1. Defibrillator, der folgendes aufweist:
- eine Hochspannungs-Generatorschaltung (1, 10, 21);
- eine Hochspannungs-Kondensatorschaltung (2, 11, 22, 40), die ausgebildet ist, um mit einer Hochspannung von der Hochspannungs-Generatorschaltung (1, 10, 21) geladen zu werden, und die aus einem metallisierten Kunststoffolienkondensator (11, 40) besteht;
- eine Ausgangsschaltung (3, 13, 23), die wenigstens einen Schalter (12) aufweist und ausgebildet ist, um die Hochspannung der Hochspannungs-Kondensatorschaltung (2, 11, 22, 40) an Paddelelektroden (4, 14, 24) anzulegen, die mit einem Körper eines Patienten in Kontakt zu bringen sind;
- eine Ladungsabnormalität-Abtastschaltung (5, 15-17, 25-27), um einen steilen Anstieg des Ladestroms und/oder einen steilen Abfall der Ladespannung zu messen, die durch einen dielektrischen Durchschlag des metallisierten Kunststoffolienkondensators (11, 40) verursacht sind;
- eine Anzeigeeinrichtung (6, 18, 28), um aufgrund eines Meßsignals der Abtastschaltung (5, 15-17, 25-27) jede Abnormalität anzuzeigen; und
- einen Schalterstromkreis (1a, 19, 21a), um aufgrund des Meßsignals die weitere Versorgung mit jeglicher Hochspannung von der Hochspannungs-Generatorschaltung (1, 10, 21) zu sperren.

2. Defibrillator nach Anspruch 1,
wobei die Ladungsabnormalität-Abtastschaltung (15-17) folgendes aufweist:
- einen Spannungsteiler (15) für die von der Hochspannungs-Kondensatorschaltung (10) zugeführte Hochspannung,
- eine Peak-Halteschaltung (16), an die eine Teilspannung des Spannungsteilers (15) angelegt wird, und
- einen Komparator (17), um die Teilspannung mit einer vorbestimmten Spannung zu vergleichen, die niedriger als die Haltespannung der Peak-Halteschaltung (16) ist, und um ein Alarm- und Sperrsignal zu erzeugen, wenn die vorhandene Teilspannung unter der vorbestimmten Spannung liegt.

3. Defibrillator nach Anspruch 1,
wobei die Ladungsabnormalität-Abtastschaltung (25-27) folgendes aufweist:
- einen A/D-Wandler (26), um eine Teilspannung der Hochspannungs-Kondensatorschaltung (22) zu digitalisieren,
- einen Speicher (27), der Daten von Kurven speichert, die den normalen Lade- und Entladebetrieb der Hochspannungs-Kondensatorschaltung (22) repräsentieren, und
- eine CPU (25), um die digitalisierte Teilspannung mit den gespeicherten Lade- und Entladekurven kontinuierlich zu vergleichen und um ein Alarm- und Sperrsignal zu erzeugen, wenn die Abweichung Normalbetriebswerte um einen vorbestimmten Betrag überschreitet.

4. Defibrillator nach einem der Ansprüche 1 bis 3,
wobei der metallisierte Kunststoffolienkondensator folgendes aufweist:
- eine Vielzahl von Kondensatorelementen (11a, 41), die jeweils aufgewickelte metallisierte Kunststoffolien (31a, 31b) und metallische Kontakte (42) haben, die an den Endflächen auf beiden Seiten davon vorgesehen, und die in einem gemeinsamen Gehäuse (45) aufgenommen sind,
- ein Paar von Kondensatoranschlüssen (11b, 47, 47a), die sich von dem Gehäuse (45) aus erstrecken und die mit den metallischen Kontaktbereichen (42) der Kondensatorelemente (11a, 41), die an den Endflächen auf deren beiden Seiten vorgesehen sind, verbunden sind, und
- Widerstände (11c, 43), die jeweils einen Widerstandswert haben, der wenigstens gleich dem Innenwiderstand der jeweiligen Kondensatorelemente (11a, 41) selbst ist, und die mit den metallischen Kontaktbereichen (42) verbunden sind, die an den Endflächen auf wenigstens einer Seite der Kondensatorelemente (11a, 41) vorgesehen sind.

5. Defibrillator nach Anspruch 4,
wobei die Widerstände (11c, 43) jeweils zwischen einen (11b, 47) des Paars von Kondensatoranschlüssen (11b, 47, 47a) und jeden der metallischen Kontaktbereiche (42) geschaltet sind, die an den Endflächen auf der einen Seite der Kondensatorelemente (11a, 41) vorgesehen sind.

6. Defibrillator nach Anspruch 4 oder 5,
wobei die metallischen Kontaktbereiche, die an den Endflächen auf der einen Seite der Kondensatorelemente vorgesehen sind, durch die Widerstände (49) der Reihe nach verbunden sind.

## Revendications

1. Défibrillateur comprenant :
- un circuit générateur haute tension (1, 10, 21) ;
- un circuit capacitif haute-tension (2, 11, 22, 40) qui est adapté pour être chargé à une tension élevée par le circuit générateur haute tension (1, 10, 21) et qui est constitué par un condensateur sous la forme de films plastiques métallisés (11, 40) ;
- un circuit de sortie (3, 13, 23) qui comporte au moins un commutateur (12) et qui est adapté pour appliquer la tension élevée du circuit capacitif haute tension (2, 11, 22, 40) à des électrodes palettes (4, 14, 24) à amener en contact avec le corps du malade ;
- un circuit détecteur d'anomalies de charge (5, 15-17, 25-27) pour détecter une brusque augmentation du courant de charge et/ou une brusque chute de la tension de charge, causées par une rupture diélectrique des films plastiques métallisés formant condensateur (11, 40) ;
- un moyen de notification (6, 18, 28) pour notifier toute anomalie en réponse à un signal de détection émis par le circuit de détection (5, 15-17, 25-27) ; et
- un interrupteur (1a, 19, 21a) pour empêcher toute fourniture ultérieure de haute tension par le circuit générateur haute-tension (1, 10, 21) en réponse à ce signal de détection.

2. Défibrillateur selon la revendication 1, dans lequel le circuit détecteur d'anomalies de charge (15-17) comprend :
- un diviseur de tension (15) pour la tension élevée fournie par le circuit capacitif haute-tension (10) ;
- un circuit de maintien de pointe (16) auquel est amenée une tension fractionnaire du diviseur de tension (15) ; et
- un comparateur (17) pour comparer cette tension fractionnaire à une tension prédéterminée inférieure à la tension de maintien du circuit de maintien de pointe (16) et pour produire une alarme et inhiber les signaux lorsque la présente tension fractionnaire est inférieure à la tension prédéterminée.

3. Défibrillateur selon la revendication 1, dans lequel le circuit détecteur d'anomalies de charge (25-27) comprend :
- un convertisseur A/N (26) pour numériser une tension fractionnaire du circuit capacitif haute tension (22) ;
- une mémoire (27) stockant les données de courbes représentant le fonctionnement normal en charge et en décharge du circuit capacitif haute tension (22) ; et
- une unité de traitement centrale (U T C; 25) pour comparer en continu la tension fractionnaire numérisée avec les courbes de charge et de décharge stockées et pour produire une alarme et inhiber les signaux si l'écart dépasse d'une valeur prédéterminée les valeurs d'un fonctionnement normal.

4. Défibrillateur selon l'une des revendications 1 à 3, dans lequel le condensateur sous forme de films plastiques métallisés comprend :
- une multiplicité d'éléments de condensateur (11a, 41) dont chacun a des films plastiques métallisés (31a, 31b) enroulés et des contacts métalliques (42) prévus sur les surfaces terminales des deux côtés des éléments de condensateur et qui sont logés dans un boîtier commun (45) ;
- deux bornes de condensateur (11b, 47, 47a) qui partent du boîtier (45) et qui sont raccordées aux portions de contact métalliques (42) des éléments de condensateur (11a, 41) prévues sur des surfaces terminales des deux côtés de ceux-ci ; et
- des résistances (11c, 43) dont chacune a une valeur de résistance au moins égale à la valeur de résistance interne des éléments de condensateur respectifs (11a, 41) et qui sont raccordées aux portions de contact métalliques (42) prévues sur les surfaces terminales sur au moins un côté des éléments de condensateur (11a, 41).

5. Défibrillateur selon la revendication 4, dans lequel chaque résistance (11c, 43) est montée entre l'une (11b, 47) de la paire de bornes de condensateur (11b, 47, 47a) et chacune des portions de contact métalliques(42) qui sont prévues sur les surfaces terminales sur un côté des éléments de condensateur (11a, 41).

6. Défibrillateur selon l'une des revendications 4 ou 5, dans lequel les portions de contact métalliques qui sont prévues sur les surfaces terminales sur un côté des éléments de condensateur sont raccordées en série par l'intermédiaire des résistances (49).
